# EUROPÄISCHE PATENTSCHRIFT

(11) **EP 2 410 940 B1**
(45) Veröffentlichungstag und Bekanntmachung des Hinweises auf die Patenterteilung: **20.11.2019**
(21) Anmeldenummer: 10709539.0
(22) Anmeldetag: 19.03.2010
(51) Int. Cl.: A61C 5/64

(54) **SPRITZE ZUR EINMALIGEN VERWENDUNG**
SYRINGE FOR ONE-TIME USE
SERINGUE A USAGE UNIQUE

(30) Priorität: 23.03.2009 EP 09155936
(43) Veröffentlichungstag der Anmeldung: 01.02.2012
(73) Patentinhaber: Sulzer Mixpac AG, 9469 Haag (CH)
(72) Erfinder: ETTLIN, Josef, CH-9453 Eichberg (CH); HEGGLIN, Armin, CH-6300 Zug (CH)
(74) Vertreter: Manitz Finsterwald Patent- und Rechtsanwaltspartnerschaft mbB
(86) Internationale Anmeldenummer: PCT/EP2010/053625
(87) Internationale Veröffentlichungsnummer: WO 2010/108868

(56) Entgegenhaltungen:
- EP-A- 0 382 481
- EP-A- 0 986 995
- EP-A- 1 616 590
- EP-A- 1 728 560
- EP-A1- 0 294 672
- EP-A1- 0 699 582
- EP-A2- 1 430 959
- DE-A1- 3 405 352
- US-A- 3 729 031

## Beschreibung

Die Erfindung betrifft eine Spritze, insbesondere eine Spritze zur Verarbeitung von einer Mehrzahl von Komponenten. Derartige Spritzen sind für die einmalige Verwendung konzipiert, das heisst sie enthalten eine Füllmasse in genau der Menge, die für eine bestimmte Anwendung üblicherweise benötigt wird. Die Spritze ist insbesondere zum gleichzeitigen Austrag von zumindest zwei Komponenten geeignet, welche vor der Verwendung gemischt werden können. Des weiteren wird ein Verfahren zum Betrieb einer Spritze offenbart.

Herkömmliche Spritzen werden zur Dosierung von kleinsten Mengen einer Füllmasse eingesetzt. Eine Spritze ist in ihrer einfachsten Ausführungsform ein Rohr mit einer Spitze. Das Rohr dient als Vorratskammer für die Füllmasse. Das Rohr mündet am Austragsende in die Spitze. Am gegenüberliegenden Ende, das als Förderende bezeichnet werden soll befindet sich ein Kolben, der innerhalb des Rohrs hin und her beweglich ist. Die Spitze enthält eine Austrittsöffnung, durch welche die Füllmasse kontinuierlich als Strahl oder diskontinuierlich in Tropfenform austreten kann. Um die Füllmasse auszutragen, verschiebt der Anwender den Kolben in Richtung der Spitze. Die Füllmasse verlässt die Spritze durch die Spitze und wird an dem vom Anwender gewünschten Ort appliziert. Für die Befüllung der Spritze mit Füllmaterial wurde bisher in der nachfolgenden Weise vorgegangen. Der Kolben wird in eine Position gebracht, welche er nach Beendung des Fördervorgangs einnimmt, nämlich die Position mit minimalem Abstand zur Spitze. Die Spitze der Spritze wird in ein Reservoir an Füllmasse eingetaucht. Währenddessen wird der Kolben von der Spitze wegbewegt, sodass Füllmasse aus dem Reservoir angesaugt wird.

Die Spritze dient dem Anwender somit in erster Linie dazu, kleine und kleinste Mengen von Füllmasse punktgenau aufzutragen. Insbesondere wenn diese Füllmasse chemisch instabil ist, kann die Füllmasse nicht in beliebiger Menge in einem Reservoir zur Verfügung gestellt werden. Das bedeutet, dass ein Bedarf besteht, derartige Spritzen dem Anwender in bereits befüllter Form zur Verfügung gestellt werden, wobei die Füllmasse in der Spritze selbst lagerfähig sein soll.

Daher gibt es Spritzen, die mittels einer Fülleinrichtung befüllt werden. Die Fülleinrichtung ist in ihrer einfachsten Form ein mit einem Reservoir verbundener Schlauch, der an das Förderende der Spritze angedockt wird. Mittels einer mit dem Schlauch verbundenen Pumpvorrichtung wird die Vorratskammer der Spritze mit Füllmasse befüllt. Nach Abschluss der Befüllung wird die Spitze der Spritze verschlossen und das Förderende der Spritze mit dem Kolben verschlossen. Die Spritze ist nunmehr vorbereitet für die Anwendung.

Wenn der Anwender die Spritze direkt vor Gebrauch befüllen möchte, steht ihm nur die Möglichkeit zur Verfügung, ein Reservoir an Füllmasse bereitzuhalten. Diese Lösung ist nachteilig, wenn für die Handhabung und Lagerung der Füllmasse besondere Vorschriften gelten, beispielsweise für Füllmassen die umweltgefährdende oder schädliche Komponenten enthalten. Einem Anwender, der derartige Füllmassen nur in geringen Mengen oder in unregelmässigen zeitlichen Abständen benötigt, ist daher darauf angewiesen, auf die Lösung der bereits befüllten Spritzen auszuweichen.

Bei Verwendung einer befüllten Spritze ist für den Anwender das Füllvolumen vorgegeben. Benötigt er für seine Anwendung jedoch verschiedene Füllvolumina, muss er demnach eine Vielzahl von befüllten Spritzen mit unterschiedlichen Füllvolumina an Lager halten. Diese Lagerhaltung kann für einen Anwender, der nur einen kleinen oder unregelmässigen Bedarf an Füllmasse problematisch sein, insbesondere bei Füllmassen, die chemisch unbeständig sind und demnach nur begrenzte Haltbarkeit aufweisen.

Aus der EP 0 699 582 A1 ist eine Kartusche und eine Spritze bekannt, wobei die Spritze über Verbindungsschläuche mit der Kartusche verbunden wird. Die EP 1 430 959 A2 betrifft eine Vorrichtung zum Mischen und Ausbringen von Mehrkomponentenmassen, bei welcher zwei Vorratskammern für unterschiedliche Komponenten vorgesehen sind. Die EP 0 382 481 A1 lehrt eine Kapsel zum Ausbringen eines Stoffes.

Es ist die Aufgabe der Erfindung, eine Lösung vorzusehen, mittels welcher der Anwender jederzeit über die gewünschte Menge an Füllmasse verfügen kann, ohne eine aufwändige Lagerhaltung betreiben zu müssen oder kostspielige Sicherheitsvorschriften zur Lagerung von gefährlichen Füllmassen einhalten zu müssen.

Die Aufgabe der Erfindung wird mit einer Spritze gemäß Anspruch 1 gelöst.

Im Unterschied zu Spritzen werden Kartuschen für die Dosierung von grösseren Mengen einer Füllmasse verwendet. Die Grenze zur Unterscheidung zwischen einer Spritze und einer Kartusche liegt bei 25 ml. Ist das Füllvolumen kleiner als 25 ml, wird eine Vorrichtung zur Dosierung desselben als Spritze bezeichnet, liegt das Füllvolumen darüber, wird die entsprechende Vorrichtung zur Dosierung als Kartusche bezeichnet.

Erfindungsgemäß enthält die Vorratskammer eine erste Teilkammer, die eine erste fliessfähige Komponente enthält und eine zweite Teilkammer, welche eine zweite fliessfähige Komponente enthält. Erfindungsgemäß kann die Spritze für die Dosierung von zwei oder mehreren fliessfähigen Komponenten eingesetzt werden.

Das Andockelement einer Spritze zur Dosierung mehrerer fliessfähiger Komponenten enthält ein erstes Eintrittsende zur Verbindung der ersten Teilkammer mit der Kartusche und ein zweites Eintrittsende zur Verbindung der zweiten Teilkammer mit der Kartusche.

Das erste Eintrittsende ist erfindungsgemäß als ein erster rohrförmiger Stutzen ausgebildet und das zweite Eintrittsende als ein zweiter rohrförmiger Stutzen ausgebildet, der zur Verbindung mit der Kartusche bestimmt ist, wobei der erste rohrförmige Stutzen einen ersten Kanal und der zweite rohrförmige Stutzen einen zweiten Kanal enthält. Der erste Kanal ist mit der ersten Teilkammer und der zweite Kanal mit der zweiten Teilkammer verbunden.

Jeder der ersten und zweiten rohrförmigen Stutzen kann ein erstes und ein zweites Dichtelement zur Aufnahme je eines ersten oder zweiten Austrittselements der Kartusche aufweisen. Insbesondere kann das Austrittselement rohrförmig ausgestaltet sein. Das Austrittselement der Kartusche kann einen ersten und zweiten Austrittskanal enthalten. Jeder der ersten und zweiten rohrförmigen Stutzen kann alternativ dazu ein erstes und ein zweites Dichtelement zur Aufnahme im Inneren eines ersten oder zweiten rohrförmigen Austrittselements der Kartusche aufweisen.

In der Vorratskammer ist ein Ausschiebeelement angeordnet, um die Füllmasse aus der Vorratskammer auszutragen.

Das Ausschiebeelement umfasst bei der Ausführung der Spritze als einer Spritze für mehrere fliessfähige Komponenten einen ersten Kolben und zumindest einen zweiten Kolben. Der erste Kolben ist in der ersten Teilkammer beweglich aufnehmbar und der zweite Kolben in der zweiten Teilkammer beweglich aufnehmbar, sodass bei Bewegung zumindest eines der ersten oder zweiten Kolben die erste und zweite fliessfähige Komponente gleichzeitig austragbar sind.

Der erste und der zweite Kolben sind mittels eines Stössels bewegbar. Der Stössel ist einstückig mit dem ersten und dem zweiten Kolben ausgebildet.

An das Austrittsende ist erfindungsgemäß ein Mischer angeschlossen. Hierbei münden die Vorratskammer oder die erste und die zweite Teilkammer am Austrittsende in einen Mischer. Der Mischer kann insbesondere als statischer Mischer ausgeführt sein. Der Einsatz eines Mischers ist insbesondere dann vorteilhaft, wenn die Spritze für eine Füllmasse verwendet wird, die aus mehreren fliessfähigen Komponenten besteht. Erfindungsgemäß ist der Mischer als Teil eines Gehäuses ausgebildet, welches die erste und zweite Teilkammer enthält. Um den Mischer herum ist ein Mischergehäuse angeordnet, welches mit dem Gehäuse verbunden ist. Die Verbindung kann dabei eine Schraubverbindung, eine Rastverbindung, eine Schnappverbindung oder eine Bayonettverbindung umfassen. Insbesondere kann das Mischergehäuse verschiebbar zu dem Mischer angeordnet sein, sodass in einer ersten Stellung des Mischergehäuses relativ zum Mischer eine Durchtrittsöffnung für die erste und zweite fliessfähige Komponente freigebbar ist, in einer zweiten Stellung diese Durchtrittsöffnung hingegen verschliessbar ist. Somit ist in diesem Ausführungsbeispiel das Mischergehäuse als verschliessbares Austrittselement ausgestaltet.

Die Vorratskammer oder die erste und zweite Teilkammer können zumindest teilweise durchsichtig sein, sodass der Füllstand kontrollierbar ist. Insbesondere besteht das Gehäuse aus einem transparenten Material, beispielsweise einem transparenten Kunststoff, sodass bei Befüllung der Spritze für den Anwender optisch erkennbar ist, wieviel Füllmasse sich bereits in der Vorratskammer befindet. In gleicher Weise ist für jede der ersten oder zweiten Teilkammern erkennbar, wie hoch der Anteil der ersten oder zweiten fliessfähigen Komponenten am Füllvolumen ist. An der Aussenseite des Gehäuses kann im Bereich der Vorratskammer oder der ersten oder zweiten Teilkammer eine Skala angebracht sein, die dem Anwender einen Hinweis darüber liefert, welches Füllvolumen die bereits eingefüllte Füllmasse enthält.

Es ist demnach auch möglich, die Spritze nur teilweise zu befüllen, falls nur ein Teil des Füllvolumens benötigt wird. Als Beispiel für eine derartige Anwendung ist beispielsweise der Auftrag eines Klebemittels oder einer Dichtmasse zu nennen. Je nach Grösse der Klebestelle oder der abzudichtenden Stelle kann die Spritze genau mit der hierzu erforderlichen Menge an Füllmasse, beziehungsweise genau mit der Mehrzahl an fliessfähigen Komponenten befüllt werden, die an der Klebestelle oder der abzudichtenden Stelle benötigt werden.

Gerade wenn die Kosten der Füllmasse ins Gewicht fallen, wie beispielsweise im Dentalbereich, ist die Möglichkeit, die Spritze präzise mit der benötigten Menge an Füllmasse zu befüllen, für den Anwender interessant. Verwendet der Anwender eine herkömmliche Spritze, die er in befülltem Zustand kauft, das heisst, die eine definierte Menge an Füllmasse enthält, muss er überschüssige Füllmasse zusammen mit der Spritze entsorgen. Neben dem Kostenfaktor bedeutet diese Variante eine Vergeudung von Füllmasse, für welche zudem zusätzliche Entsorgungskosten anfallen können.

Mit den vorhin beschriebenen Ausführungsbeispielen steht dem Anwender jedoch eine Kartusche zur Verfügung, welche die Füllmasse in einer Menge enthält, die ein Mehrfaches des Füllvolumens der Spritze beträgt, aber wesentlich handlicher als die Installation einer eigenen Abfüllanlage ist, Insbesondere für Anwender mit geringem oder unregelmässigem Bedarf an Füllmasse ist eine derartige Abfüllanlage nicht geeignet, da sie nicht kontinuierlich arbeiten kann und somit nicht wirtschaftlich für den vorhin beschriebenen Fall einsetzbar ist.

Die Kartusche hat beispielsweise ein Füllvolumen, welches im Bereich des 5 bis 50-fachen des Füllvolumens der Spritze liegt, bevorzugt im Bereich 5 bis 40- fache des Füllvolumens der Spritze liegt, besonders bevorzugt im Bereich des 5 bis 25-fachen des Füllvolumens liegt.

Benötigt der Anwender nun eine definierte Menge an Füllmasse, verbindet er die Kartusche mit dem Andockelement der Spritze und befüllt die Spritze nach dem nachgehend beschriebenen Verfahren:
Das Verfahren zum Betrieb einer Spritze, insbesondere nach einem der vorhergehenden Ausführungsbeispiele umfasst die Schritte des Befüllens der Spritze mit einer Füllmasse sowie des Austrags der Füllmasse, wobei das Befüllen die nachfolgenden Schritte umfasst:
Andocken der Spritze an eine Kartusche durch Verbinden eines an einem Förderende der Spritze angeordneten Eintrittsendes einer Vorratskammer mit einem Austrittselement der Kartusche,
Öffnen einer Entlüftungsöffnung, sodass Luft aus der Vorratskammer entweichen kann,
Einbringen der Füllmasse in die Vorratskammer und
Schliessen der Entlüftungsöffnung sobald die Vorratskammer mit Füllmasse befüllt ist,
Verschliessen der befüllten Vorratskammer mittels eines verschliessbaren Austrittselements am Austrittsende,
Verschliessen der befüllten Vorratskammer mittels eines Ausschiebeelements am Förderende,
Der Austrag der Füllmasse umfasst die nachfolgenden Schritte:
   Öffnen des verschliessbaren Austrittselements der befüllten Vorratskammer

Austragen der Füllmasse, indem sie in der Vorratskammer mit Druck beaufschlagt wird, wozu das Ausschiebeelement derart verschoben wird, dass das Füllvolumen in der Vorratskammer abnimmt.

Soll die Spritze für mehrere fliessfähige Komponenten betrieben werden, werden während des Befüllens eine erste fliessfähige und eine zweite fliessfähige Komponente in eine erste Teilkammer und eine zweite Teilkammer eingebracht. Während des Austragens treten die erste fliessfähige und die zweite fliessfähige Komponente aus der ersten und zweiten Teilkammer aus, wobei jeder der ersten und zweiten Kolben von einem beweglichen Stössel unter Ausüben einer Druckkraft derart in der entsprechenden ersten oder zweiten Teilkammer verschoben wird, dass das Füllvolumen in jeder der ersten und zweiten Teilkammern abnimmt.

Die ersten und zweiten fliessfähigen Komponenten werden nach dem Austreten aus der ersten und zweiten Teilkammer gemischt.

Nachfolgend wird die Erfindung anhand der Zeichnungen erläutert. Es zeigen:
Fig. 1 eine Ansicht auf eine Spritze gemäss eines Ausführungsbeispiels, das nicht unter Anspruch 1 fällt
Fig. 2 eine Ansicht auf eine Spritze gemäss eines zweiten Ausführungsbeispiels der Erfindung
Fig. 3 eine Ansicht auf eine Spritze gemäss des zweiten Ausführungsbeispiels, wobei die Spritze auf der Kartusche aufgesetzt ist
Fig. 4 einen Schnitt durch eine Kartusche mit aufgesetzter Spritze.
Fig. 5 eine Ansicht auf eine Spritze gemäss eines dritten Ausführungsbeispiels der Erfindung

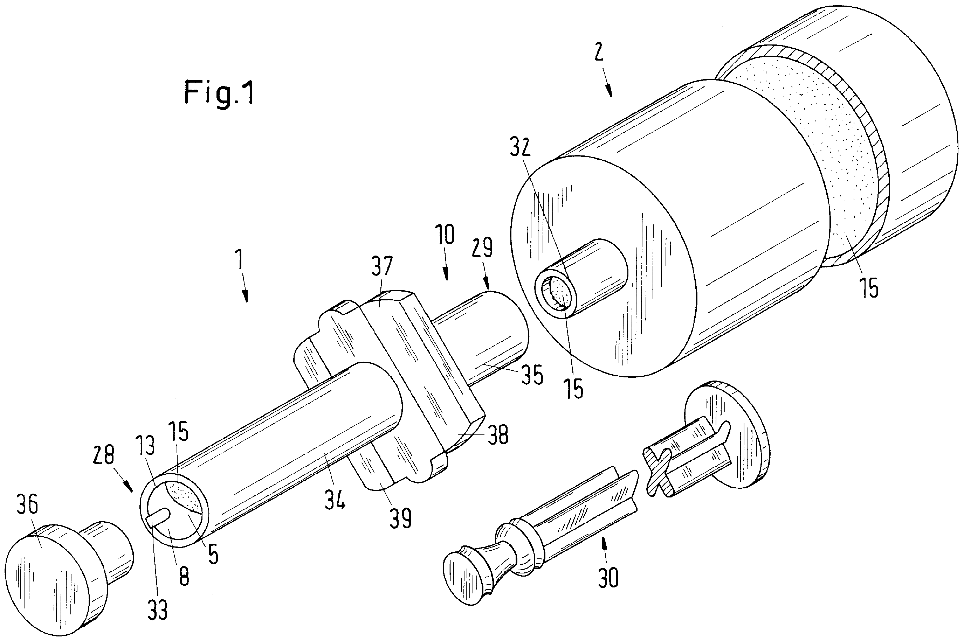

Fig. 1 zeigt ein Ausführungsbeispiel der Spritze 1, das nicht unter Anspruch 1 fällt, welche zur einmaligen Dosierung einer Füllmasse 15, also zur einmaligen Verwendung, bestimmt ist. Insbesondere wird eine derartige Spritze zur Dosierung kleiner und kleinster Mengen der Füllmasse 15 verwendet. Die Spritze 1 enthält eine Vorratskammer 5 zur Aufnahme der Füllmasse 15. Die Vorratskammer 5 weist ein Austrittsende 28 zum Austrag der Füllmasse 15 und ein Förderende 29 auf, welches dem Austrittsende 28 gegenüber liegt. Die Vorratskammer 5 erstreckt sich somit gemäss Fig. 1 in dem rohrförmigen Abschnitt zwischen Förderende und Austrittsende. Die Vorratskammer 5 ist von einem Gehäuse 34 umgeben, sodass die Füllmasse 15 in der Vorratskammer 5 aufgenommen werden kann. Damit die Füllmasse nicht unkontrolliert aus der Vorratskammer 5 austreten kann, enthält die Vorratskammer 5 ein verschliessbares Austrittselement 13 sowie ein Verschlusselement auf der Förderseite. Das Verschlusselement ist als Ausschiebeelement 30 dargestellt. Wenn das verschliessbare Austrittselement 13 in seiner geschlossenen Stellung ist und das Ausschiebeelement in das Eintrittsende 35 eingesetzt ist, ist die Füllmasse 15 in der Vorratskammer 5 zumindest für eine begrenzte Zeitdauer lagerfähig. Das verschliessbare Austrittselement 13 kann einen Stopfen 36 umfassen, der das Austrittsende 28 dichtend verschliessen kann, indem er fluiddicht an der inneren Oberfläche des verschliessbaren Austrittselements 13 aufliegt. Das verschliessbare Austrittselement 13 kann auch ein Innengewinde umfassen, in welchen ein mit einem entsprechenden Aussengewinde versehener Stopfen aufnehmbar ist.

Das verschliessbare Austrittselement 13 kann auch mit dem Stopfen 36 eine Zwischenstellung einnehmen, in welcher das Austrittsende 28 nicht vollständig geöffnet ist. In dieser Zwischenstellung ist die Entlüftungsöffnung 32 offen, sodass Luft aus der Vorratskammer 5 ungehindert austreten kann. Nach einem anderen Ausführungsbeispiel kann die Entlüftungsöffnung auch durch das Austrittsende selbst gebildet werden. Alternativ dazu könnte eine verschliessbare Entlüftungsöffnung oder ein Entlüftungsventil auch im Stopfen angebracht sein, was nicht in der Darstellung gezeigt ist.

Die Füllmasse ist in der Vorratskammer 5 lagerfähig wenn das verschliessbare Austrittselement 13 sich in seiner Schliessstellung befindet und das Eintrittsende 35 ebenfalls durch das Ausschiebeelement 30 verschlossen ist.

Ein Andockelement 10 ist zur Verbindung der Vorratskammer 5 mit einer Kartusche 2 zum Befüllen der Vorratskammer 5 mit der Füllmasse 15 vorgesehen. Das Andockelement 10 kann beispielsweise als eine Verlängerung des Gehäuses 34 ausgebildet sein, welche das Eintrittsende 35 umfasst. Das vom Eintrittsende 35 eingeschlossene Volumen gehört nicht zum Füllvolumen, sondern dient der Aufnahme eines Austrittselements 32 der Kartusche. Der zwischen dem Eintrittsende 35 und der Vorratskammer 5 gezeichnete Vorsprung 37 stellt die eintrittseitige Begrenzung der Vorratskammer 5 dar. Der Vorsprung 37 ist als Griff ausgebildet. Dieser Griff hat zwei Auflageflächen 38, 39 für je einen Finger. Diese Auflageflächen 38, 39 werden benutzt, um beim Entleeren der Spritze 1 mit zwei Fingern einen Gegendruck zu dem mit dem Daumen gehaltenen Ausschiebeelement 30 aufbauen zu können.

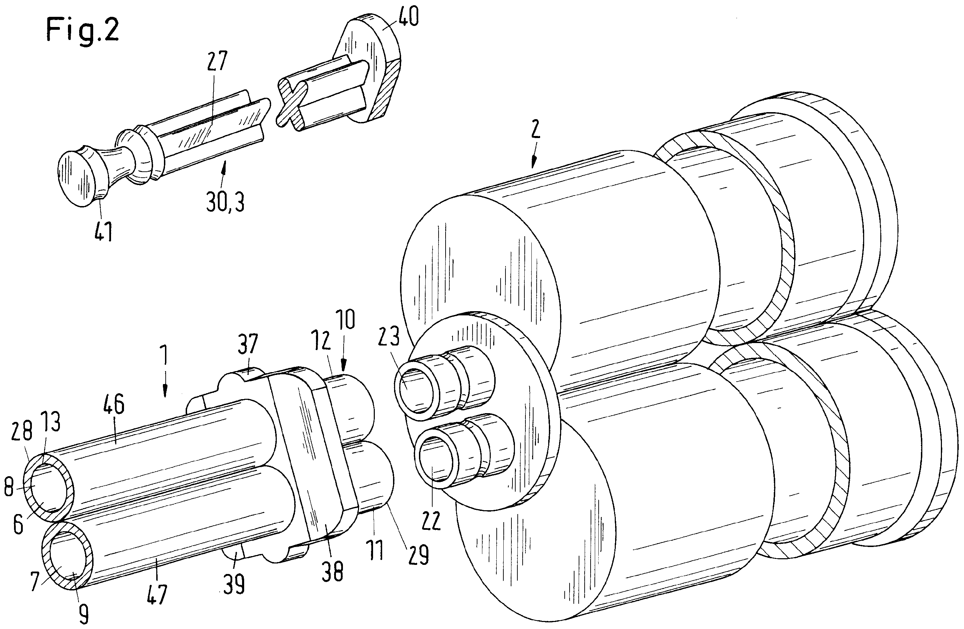

Fig. 2 zeigt ein Ausführungsbeispiel der erfindungsgemässen Spritze 1 für mehrere Komponenten, welche zur einmaligen Verwendung bestimmt ist. Die Spritze 1 umfasst eine erste Teilkammer 6 für eine erste Komponente 8, eine zweite Teilkammer 7 für eine zweite Komponente 9. Die erste Teilkammer 6 ist getrennt von der zweiten Teilkammer 7, sodass die beiden Komponenten nicht miteinander in Kontakt kommen. Derartige Komponenten interagieren zumeist miteinander, sobald sie miteinander in Berührung kommen, wobei chemische Reaktionen ablaufen können. Die Interaktion der Komponenten ist zumeist der Effekt, welcher in einer Anwendung benötigt wird, allerdings ist diese Interaktion unerwünscht, solange die Komponenten nicht im Rahmen der für sie bestimmten Anwendung eingesetzt werden.

Vor dem Gebrauch muss die Spritze oftmals gelagert und transportiert werden, und zwar teilweise in befülltem Zustand, der nachfolgend als Lagerzustand bezeichnet wird. Für die gesamte Dauer des Lagerzustandes muss sichergestellt sein, dass die beiden Komponenten 8,9 nicht miteinander in Berührung kommen.

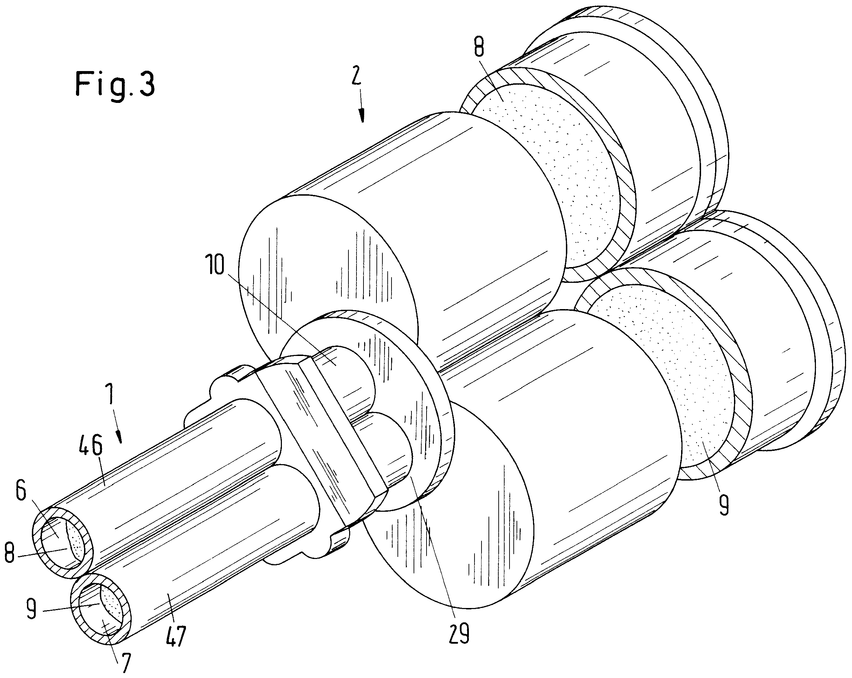

Das Ausführungsbeispiel gemäss Fig. 2 oder Fig. 3 unterscheidet sich unter anderem vom Ausführungsbeispiel gemäss Fig. 1, dass das Andockelement 10 ein erstes Eintrittsende 11 zur Verbindung der ersten Teilkammer 6 mit der Kartusche 2 enthält, und ein zweites Eintrittsende 12 zur Verbindung der zweiten Teilkammer 7 mit der Kartusche 2 enthält. Das erste Eintrittsende 11 ist als ein erster rohrförmiger Stutzen 16 ausgebildet und das zweite Eintrittsende 12 ist als ein zweiter rohrförmiger Stutzen 17 ausgebildet, der zur Verbindung mit der Kartusche bestimmt ist, wobei der erste rohrförmige Stutzen 16 einen ersten Kanal 18 und der zweite rohrförmige Stutzen 17 einen zweiten Kanal 19 enthalten. Der erste Kanal ist mit der ersten Teilkammer 6 und der zweite Kanal 19 mit der zweiten Teilkammer 7 verbunden.

Jeder der ersten und zweiten rohrförmigen Stutzen 16, 17 kann ein erstes und ein zweites Dichtelement 24, 25 zur Aufnahme je eines ersten oder zweiten Austrittselements 22, 23 der Kartusche 2 aufweisen. Jedes der Austrittselemente 22, 23 der Kartusche kann somit ebenfalls als rohrförmiger Stutzen ausgebildet sein. Derartige Kartuschen sind beispielsweise in der EP 0 730 913 gezeigt.

Wie in Fig. 1 kann in jeder der Teilkammern 6,7 ein Ausschiebeelement 30 (siehe Fig. 1) angeordnet sein, um die entsprechende fliessfähige Komponente 8,9 aus der Teilkammer 6,7 auszutragen. Das Ausschiebeelement 30 besteht in Fig. 2 aus einem ersten Kolben 3 und einen zweiten Kolben 4. In Fig. 2 ist nur der Kolben 3 gezeigt, der zur Aufnahme in der Teilkammer 6 vorgesehen ist.

Der erste Kolben 3 ist in der ersten Teilkammer 6 beweglich aufnehmbar und der zweite Kolben 4 ist in der zweiten Teilkammer 7 beweglich aufnehmbar, sodass bei Bewegung zumindest eines der ersten oder zweiten Kolben 3,4 die erste und zweite fliessfähige Komponente 8,9 gleichzeitig austragbar sind. Hierzu sind der erste Kolben 3 und der zweite Kolben 4 und der Stössel 27 einstückig ausgebildet.

Der erste und der zweite Kolben 3, 4 weisen mindestens ein Dichtungselement 41 auf, welches insbesondere als Dichtungslippe ausgebildet sein kann.

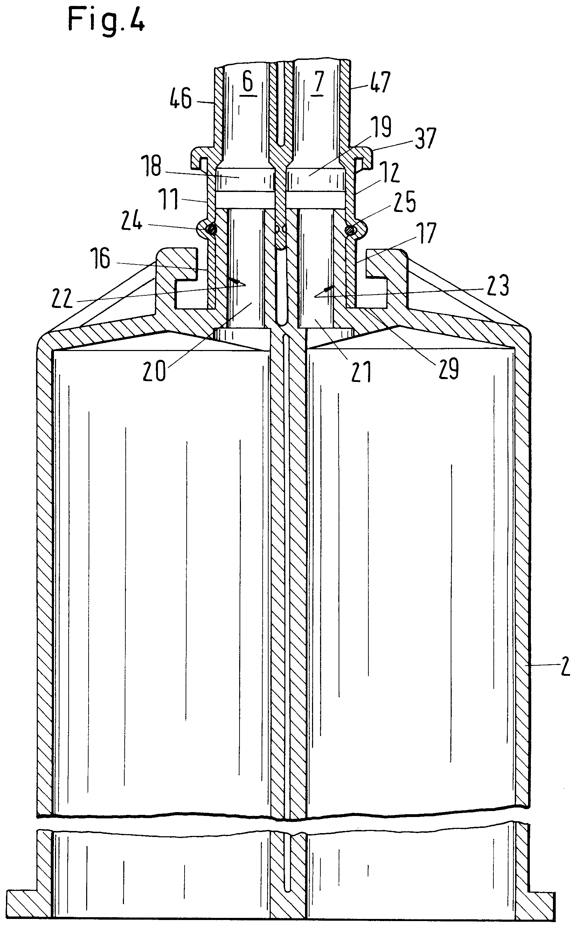

Fig. 4 zeigt einen Schnitt durch eine Kartusche mit aufgesetzter Spritze. Von der Spritze ist nur das am Förderende 29 befindliche erste und zweite Eintrittsende 11, 12 gezeigt, an welches die Teilkammern 6,7 anschliessen. Das erste Eintrittsende 11 ist als erster rohrförmiger Stutzen 16 ausgebildet, das zweite Eintrittsende 12 als zweiter rohrförmiger Stutzen 17 ausgebildet. In den ersten rohrförmigen Stutzen 16 greift ein erstes Austrittselement 22 der Kartusche 2 ein, in den zweiten rohrförmigen Stutzen 17 greift ein zweites Austrittselement 23 der Kartusche 2 ein. Das erste Austrittselement 23 enthält einen ersten Austrittskanal 20 und das zweite Austrittselement einen zweiten Austrittskanal 21. Zwischen jedem der rohrförmigen Stutzen 16, 17 und dem entsprechenden Austrittselement 22, 23 ist je ein Dichtelement 24, 25 angeordnet.

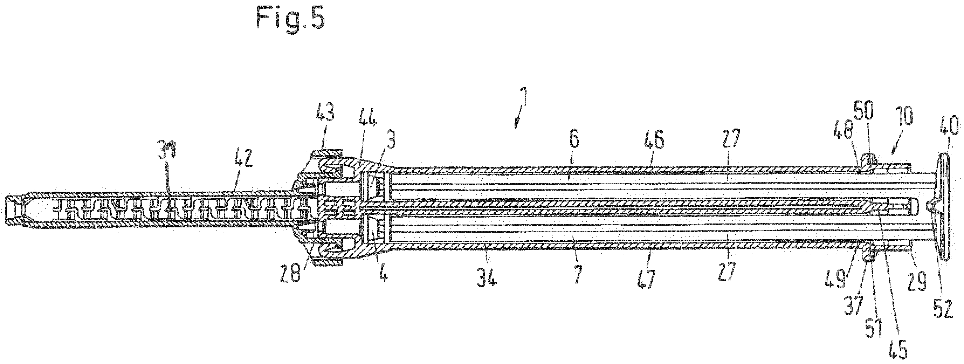

Fig. 5 zeigt ein Ausführungsbeispiel einer Spritze gemäss der Erfindung. Die Spritze 1 wird zum gleichzeitigen Austrag einer ersten Komponente 8 und einer zweiten Komponente 9 verwendet. Die erste und zweite Komponente 8,9 wird durch Verbinden je eines Austrittselements 22, 23 einer Kartusche (siehe Fig. 4) in die erste Teilkammer 6 und die zweite Teilkammer 7 eingefüllt. Die Spritze kann nach Bedarf teilweise oder auch vollständig befüllt werden. Wenn die Spritze befüllt wird, ist das verschliessbare Austrittselement in seiner geöffneten Stellung. Erfindungsgemäß wird das verschliessbare Austrittselement 13 durch ein Mischergehäuse 42 gebildet. Der Mischer 31 ist in dem Mischergehäuse 42 angeordnet und ist einstückig mit dem Gehäuse 34 ausgeführt. Der Mischer 31 ist insbesondere als statischer Mischer ausgestaltet. Das Mischergehäuse 42 enthält je ein entsprechendes Dichtelement, mittels welchem die korrespondierende Austrittsöffnung am Austrittsende 28 der Spritze verschliessbar ist.

Das Mischergehäuse 42 enthält ein Kopplungselement 43, welches zum Eingriff mit dem Gehäuse 34 bestimmt ist. Das Kopplungselement 43 ist von einem Eingriffselement 44 aufgenommen, welches das Austrittsende 28 umgibt. Das Eingriffselement 44 ist als Teil des Gehäuses 34 ausgeführt. Das Kopplungselement 43 kann relativ zum Eingriffselement 44 verschoben werden, sodass das Mischergehäuse relativ zum Mischer und zum Austrittsende 28 entweder in einer geschlossenen oder offenen Stellung haltbar ist. Das Mischergehäuse 42 wird beispielsweise während des Befüllens in einer offenen Stellung gehalten, damit Luft, welche in der ersten oder zweiten Teilkammer 6,7 vorhanden ist, über Austrittsöffnungen, die zum Austrittsende 28 führen, entweichen kann. Das Mischergehäuse 42 wird insbesondere so lange in seiner offenen Stellung gehalten, solange die Befüllung vorgenommen wird, um zu vermeiden, dass sich in der ersten oder zweiten Teilkammer 6,7 ein Druck aufbaut, der ein fortgesetztes Befüllen erschweren würde. Ist die Befüllung abgeschlossen, wird das Mischergehäuse 42 in seine geschlossene Stellung bewegt, in welcher die Austrittsöffnungen am Austrittsende 28 verschlossen gehalten werden. Auf der Förderseite 29 werden die erste und zweite Teilkammer 6,7 mit einem ersten und einem zweiten Kolben 3,4 verschlossen.

Der erste und zweite Kolben 3,4 sind mittels eines Stössels 27 bewegbar, um die beiden Komponenten 8,9 gleichzeitig auszutragen. Der Stössel 27 ist mit den Kolben 3,4 einstückig verbunden. Zu Beginn des Austrags wird das Mischergehäuse 42 von seiner geschlossenen Stellung in die offene Stellung bewegt. In dieser Stellung stehen die Austrittsöffnungen am Austrittsende mit dem Mischraum, der sich im Inneren des Mischergehäuses erstreckt. Die erste und die zweite Komponente 8, 9 sowie allfällige Luft können in den Mischer eingetragen werden. Die Luft entweicht vorab durch die Austrittsöffnung des Mischergehäuses. Anschliessend erfolgt die Durchmischung der ersten und zweiten Komponente 8, 9 durch den Mischer 31. Für Luft, welche zwischen dem ersten oder zweiten Kolben 3, 4 und der Füllmasse eingeschlossen ist, können am entsprechenden Kolben oder an der Innenwand der entsprechenden Teilkammer Entlüftungsbohrungen oder Entlüftungsnuten vorgesehen sein, die in Fig. 5 nicht dargestellt sind.

Die erste Teilkammer 6 und die zweite Teilkammer 7 sind als erste und zweite Rohre 46, 47 ausgebildet, wobei die beiden Rohre 46, 47 durch das Andockelement 10 miteinander verbunden sind. Zumindest eines der Rohre 46, 47 kann einen Bereich 48, 49 aufweisen, welcher einen kleineren Innendurchmesser als das entsprechende Rohr 46, 47 aufweist. Dieser Bereich kann insbesondere als Absatz oder als Rille ausgestaltet sein. An diesem Bereich stehen der erste und zweite Kolben 3,4 an, sodass der Anwender beim Ausbringen der Füllmasse an dieser Stelle einen Widerstand verspürt. Dieser Widerstand zeigt dem Anwender an, dass ab diesem Zeitpunkt der Austrag der Füllmasse beginnt. Spätestens zu diesem Zeitpunkt muss auch der Mischer 31 derart bewegt werden, dass das Austrittsende 28 der Spritze derart mit dem Mischer verbunden ist, dass ein Eintritt der entsprechenden Komponente 8, 9 der Füllmasse 15 in den Mischer möglich ist und eine entsprechende Durchtrittsöffnung offen ist, sodass die Komponenten 8, 9 in den Mischer 31 eintreten können.

Alternativ oder in Ergänzung hierzu kann zumindest einer der rohrförmigen Stutzen 16, 17 einen Bereich 50, 51 aufweisen, welcher einen kleineren Innendurchmesser als der entsprechende rohrförmige Stutzen 16, 17 aufweist.

Das Andockelement 10 weist einen Vorsprung 37 auf, welcher die beiden Teilkammern 6, 7 miteinander verbindet. Das Stegelement 45 schliesst an den Vorsprung 37 an und erstreckt sich in Richtung der rohrförmigen Stutzen 16, 17. Das Stegelement 45 kann kürzer als die rohrförmigen Stutzen sein. Das Stegelement 45 kann in eine Ausnehmung münden, die sich vom Förderende 29 in Richtung des Vorsprungs 37 erstreckt. Diese Ausnehmung kann dazu dienen, einen am Stössel befindlichen Verbindungssteg 52 aufzunehmen.

Zumindest eine der Vorratskammern 5,6,7 kann nach jedem der Ausführungsbeispiele zumindest teilweise durchsichtig sein, sodass der Füllstand der Füllmasse 8,9,15 in der entsprechenden Vorratskammer 5,6,7 kontrollierbar ist.

Der Betrieb der Spritze 1 umfasst die Schritte des Befüllens der Spritze 1 mit einer Füllmasse 8,9,15 sowie des Austrags der Füllmasse.

Wird die Spritze 1 nach einem der vorhergehenden Ausführungsbeispiele befüllt, umfasst das Befüllen die nachfolgenden Schritte:
Andocken der Spritze 1 an eine Kartusche 2 durch Verbinden eines an einem Förderende 29 der Spritze 1 angeordneten Eintrittsendes 11,12 einer Vorratskammer 5,6,7 mit einem Austrittselement 22, 23, 32 der Kartusche 2,
Öffnen einer Entlüftungsöffnung 33, sodass Luft aus der Vorratskammer 5,6,7 entweichen kann,
Einbringen der Füllmasse 8,9,15 in die Vorratskammer 5,6,7 und
Schliessen der Entlüftungsöffnung 33 sobald die Vorratskammer 5,6,7 mit Füllmasse 8,9,15 befüllt ist,
Verschliessen der befüllten Vorratskammer 5,6,7 mittels eines verschliessbaren Austrittselements 13 am Austrittsende 28,
Verschliessen der befüllten Vorratskammer 5,6,7 mittels eines Ausschiebeelements 3,4,30 am Förderende 29.

Insbesondere kann als Entlüftungsöffnung 33 auch die Austrittsöffnung für die Füllmasse am Austrittsende 28 der Spritze gemeint sein. Insbesondere wenn der Fortschritt der Befüllung zu jedem Zeitpunkt sichtbar ist, da das Gehäuse durchsichtig ist, das heisst, aus transparentem Material gefertigt ist oder zumindest Öffnungen aufweist, die transparentes Material enthalten, kann der Anwender zu jeder Zeit den Grad der Befüllung feststellen und somit sicher vermeiden, dass Füllmasse das Austrittsende 28 vorzeitig verlässt. Alternativ oder in Ergänzung dazu kann das verschliessbare Austrittselement 13 Entlüftungsöffnungen enthalten oder in Kombination mit dem Gehäuse 34 eine Entlüftungsöffnung ausbilden. Die Grösse der Entlüftungsöffnung 33 kann einstellbar sein, beispielsweise indem eine Kombination eines verschliessbaren Austrittselements 13 mit dem Gehäuse 34 vorgesehen ist, welche mindestens eine konische Oberfläche aufweist. Der Abstand zwischen dem verschliessbaren Austrittselement 13 und dem Gehäuse 34 im Bereich der konischen Oberfläche kann dergestalt ausgestaltet sein, dass im geschlossenen Zustand die konische Oberfläche die Öffnung fluiddicht verschliesst, in einem teilweise geöffneten Zustand einen Austritt einer geringen Luftmenge ermöglicht und in vollständig geöffneter Stellung den Austritt einer grossen Luftmenge erlaubt oder den Austritt der Füllmasse ermöglicht.

Alternativ hierzu oder in Ergänzung hierzu kann eine Entlüftungsöffnung 33 am Kolben 3,4 vorgesehen sein. Die Entlüftungsöffnung kann in diesem Fall eine Membran umfassen, welche unter Druck eine Öffnung zum Austritt von Luft freigibt, oder ein Entlüftungsventil, welches unter Druck oder unter Auflage des Stössels öffnet. Alternativ dazu kann an der Innenwand des Gehäuses oder im Mantelbereich des Kolbens eine Öffnung oder eine Nut vorgesehen sein, welche ein Austreten von Luft zwischen dem Mantelbereich des Kolbens und der Innenwand des Gehäuses verhindert.

Der Austrag der Füllmasse 8,9,15 umfasst die nachfolgenden Schritte: Öffnen des verschliessbaren Austrittselements 13 der befüllten Vorratskammer 5,6,7
Austragen der Füllmasse 8,9,15, indem sie in der Vorratskammer 5,6,7 mit Druck beaufschlagt wird, wozu das Ausschiebeelement 3,4,30 derart verschoben wird, dass das Füllvolumen in der Vorratskammer 5,6,7 abnimmt.

Zumindest zu Beginn des Austrags der Füllmasse kann die Entlüftungsöffnung welche sich in geöffnetem Zustand befindet, ermöglichen, dass Luft, welche noch zwischen Füllmasse und Kolben eingeschlossen ist, entweichen kann.

Während des Befüllens kann eine erste fliessfähige und eine zweite fliessfähige Komponente 8,9 in eine erste Teilkammer 6 und eine zweite Teilkammer 7 eingebracht werden und während des Austragens die erste und die zweite fliessfähige Komponente 8,9 aus der ersten und zweiten Teilkammer 6,7 austreten, wobei jeder der ersten und zweiten Kolben 3,4 von einem beweglichen Stössel 27 unter Ausüben einer Druckkraft derart in der entsprechenden ersten oder zweiten Teilkammer 6,7 verschoben wird, dass das Füllvolumen in jeder der ersten und zweiten Teilkammern 6,7 abnimmt.

## Patentansprüche

1. Spritze (1) zur einmaligen Dosierung einer Füllmasse (15), mit
einer Vorratskammer (5,6,7) zur Aufnahme der Füllmasse (8,9,15), wobei die Vorratskammer (5,6,7) ein Austrittsende (28) zum Austrag der Füllmasse (8,9,15) und ein Förderende (29) aufweist, welches dem Austrittsende (28) gegenüber liegt, wobei die Vorratskammer (5,6,7) ein verschliessbares Austrittselement (13) enthält, sodass die Füllmasse (8,9,15) in der Vorratskammer (5,6,7) lagerfähig ist, wobei die Vorratskammer (5,6,7) eine erste Teilkammer (6), die eine erste fliessfähige Komponente (8) enthält, und eine zweite Teilkammer (7), die eine zweite fliessfähige Komponente (9) enthält, aufweist, wobei die Vorratskammer (5,6,7) von einem Gehäuse (34) umgeben ist,
einem Mischer (31) und einem Mischergehäuse (42), die am Austrittsende (28) angebracht sind, wobei das Mischergehäuse das verschliessbare Austrittselement (13) bildet, wobei der Mischer (31) einstückig mit dem Gehäuse (34), das die erste und zweite Teilkammer (6,7) enthält, ausgebildet ist und das Mischergehäuse (42) mit dem Gehäuse (34) verbunden ist und
einem Andockelement (10) zur Verbindung der Vorratskammer (5,6,7) mit einer Kartusche (2) zum Befüllen der Vorratskammer (5,6,7) mit der Füllmasse (8,9,15), wobei das Andockelement (10) ein erstes Eintrittsende (11) zur Verbindung der ersten Teilkammer (6) mit der Kartusche (2) und ein zweites Eintrittsende (12) zur Verbindung der zweiten Teilkammer (7) mit der Kartusche (2) enthält, wobei das erste Eintrittsende (11) als ein erster rohrförmiger Stutzen (16) ausgebildet ist und das zweite Eintrittsende (12) als ein zweiter rohrförmiger Stutzen (17) ausgebildet ist, der zur Verbindung mit der Kartusche (2) bestimmt ist, wobei das Andockelement (10) einen Vorsprung (37) aufweist, der die erste und die zweite Teilkammer (6, 7) miteinander verbindet, wobei der erste rohrförmige Stutzen (16) einen ersten Kanal (18) und der zweite rohrförmige Stutzen (17) einen zweiten Kanal (19) enthält, wobei der erste Kanal mit der ersten Teilkammer (6) und der zweite Kanal (19) mit der zweiten Teilkammer (7) verbunden ist,
wobei die Spritze (1) ein in der Vorratskammer (5,6,7) aufnehmbares Ausschiebeelement (3,4,30) aufweist, um die Füllmasse aus der Vorratskammer (5,6,7) auszutragen,
wobei das Ausschiebeelement (30) mindestens einen ersten Kolben (3) und einen zweiten Kolben (4) umfasst, wobei der erste Kolben (3) in der ersten Teilkammer (6) beweglich aufnehmbar ist und der zweite Kolben (4) in der zweiten Teilkammer (7) beweglich aufnehmbar ist, sodass bei Bewegung zumindest eines der ersten oder zweiten Kolben (3,4) die erste und zweite fliessfähige Komponente (8,9) gleichzeitig austragbar sind, und
wobei ein Stössel (27) vorgesehen ist, wobei der erste und der zweite Kolben (3,4) mittels des Stössels (27) bewegbar sind und der Stössel (27) mit dem ersten Kolben (3) und dem zweiten Kolben (4) einstückig ausgebildet ist.

2. Spritze (1) nach Anspruch 1, wobei der erste und zweite rohrförmige Stutzen (16, 17) über ein Stegelement (45) miteinander verbunden sind.

3. Spritze (1) nach einem der vorhergehenden Ansprüche, wobei die erste Teilkammer (6) und die zweite Teilkammer (7) als erste und zweite Rohre (46, 47) ausgebildet sind, wobei die beiden Rohre (46, 47) durch das Andockelement (10) miteinander verbunden sind.

4. Spritze (1) nach Anspruch 3, wobei zumindest eines der Rohre (46, 47) einen Bereich (48, 49) aufweist, welcher einen kleineren Innendurchmesser als das entsprechende Rohr (46, 47) aufweist.

5. Spritze (1) nach einem der Ansprüche 1 bis 4, wobei zumindest einer der rohrförmigen Stutzen (16, 17) einen Bereich (50, 51) aufweist, welcher einen kleineren Innendurchmesser als der entsprechende rohrförmige Stutzen (16, 17) aufweist.

6. Spritze (1) nach Anspruch 2, wobei das Stegelement (45) an den Vorsprung (37) anschliesst und sich in Richtung der rohrförmigen Stutzen (16, 17) erstreckt.

7. Spritze (1) nach einem der vorhergehenden Ansprüche, wobei die Vorratskammer (5,6,7) zumindest teilweise durchsichtig ist, sodass der Füllstand der Füllmasse (8,9,15) in der Vorratskammer (5,6,7) kontrollierbar ist.

8. Ausrüstung zur Dosierung einer Füllmasse (8,9,15), umfassend eine Kartusche (2) und zumindest eine Spritze (1) nach einem der vorhergehenden Ansprüche, wobei die Kartusche die Füllmasse (8, 9, 15) enthält und ein Austrittselement (22, 23) an der Kartusche vorgesehen ist, welches zum Eingriff in das Andockelement (10) vorgesehen ist, um die Vorratskammer (5, 6, 7) mit der Füllmasse (8, 9, 15) aus der Kartusche zu befüllen.

## Claims

1. A syringe (1) for a single metering of a filler material (15), comprising
a supply chamber (5, 6, 7) for receiving the filler material (8, 9, 15), wherein the supply chamber (5, 6, 7) has a discharge end (28) for dispensing the filler material (8, 9, 15) and a conveying end (29) which is disposed opposite the discharge end (28), wherein the supply chamber (5, 6, 7) includes a closable discharge element (13) so that the filler material (8, 9, 15) is storable in the supply chamber (5, 6, 7), wherein the supply chamber (5, 6, 7) includes a first part chamber (6) which includes a first flowable component (8) and a second part chamber (7) which includes a second flowable component (9), and wherein the supply chamber (5, 6, 7) is surrounded by a housing (34);
a mixer (31) and a mixer housing (42) which are attached to the discharge end (28), wherein the mixer housing forms the closable discharge element (13), and wherein the mixer (31) is formed in one piece with the housing (34) which includes the first and second part chambers (6, 7) and the mixer housing (42) is connected to the housing (34); and
a docking element (10) for connecting the supply chamber (5, 6, 7) to a cartridge (2) for filling the supply chamber (5, 6, 7) with the filler material (8, 9, 15), wherein the docking element (10) includes a first inlet end (11) for connecting the first part chamber (6) to the cartridge (2) and a second inlet end (12) for connecting the second part chamber (7) to the cartridge (2), wherein the first inlet end (11) is configured as a first tubular stub (16) and the second inlet end (12) is configured as a second tubular stub (17) which is intended for the connection to the cartridge (2), wherein the docking element (10) has a projection (37) which connects the first and second part chambers (6, 7) to one another, wherein the first tubular stub (16) includes a first passage (18) and the second tubular stub (17) includes a second passage (19), and wherein the first passage is connected to the first part chamber (6) and the second passage (19) is connected to the second part chamber (7),
wherein the syringe (1) has an expulsion element (3, 4, 30) receivable in the supply chamber (5, 6, 7) to dispense the filler material from the supply chamber (5, 6, 7);
wherein the expulsion element (30) comprises at least one first piston (3) and a second piston (4), with the first piston (3) being movably receivable in the first part chamber (6) and the second piston (4) being movably receivable in the second part chamber (7) so that, on the movement of at least one of the first or second pistons (3, 4), the first and second flowable components (8, 9) can be dispensed simultaneously; and
wherein a plunger (27) is provided, with the first and second pistons (3, 4) being movable by means of the plunger (27) and the plunger (27) being formed in one piece with the first piston (3) and with the second piston (4).

2. A syringe (1) in accordance with claim 1, wherein the first and second tubular stubs (16, 17) are connected to one another via a web element (45).

3. A syringe (1) in accordance with one of the preceding claims, wherein the first part chamber (6) and the second part chamber (7) are configured as first and second tubes (46, 47), with the two tubes (46, 47) being connected to one another by the docking element (10).

4. A syringe (1) in accordance with claim 3, wherein at least one of the tubes (46, 47) has a region (48, 49) which has a smaller inner diameter than the corresponding tube (46, 47).

5. A syringe (1) in accordance with any one of the claims 1 to 4, wherein at least one of the tubular stubs (16, 17) has a region (50, 51) which has a smaller inner diameter than the corresponding tubular stub (16, 17).

6. A syringe (1) in accordance with claim 2, wherein the web element (45) adjoins the projection (37) and extends in the direction of the tubular stubs (16, 17).

7. A syringe (1) in accordance with any one of the preceding claims, wherein the supply chamber (5, 6, 7) is at least partly transparent so that the filling level of the filler material (8, 9, 15) in the supply chamber (5, 6, 7) can be monitored.

8. Equipment for the metering of a filler material (8, 9, 15) comprising a cartridge (2) and at least one syringe (1) in accordance with any one of the preceding claims, wherein the cartridge includes the filler material (8, 9, 15) and a discharge element (22, 23) is provided at the cartridge which is intended for engagement into the docking element (10) to fill the supply chamber (5, 6,7) with the filler material (8, 9, 15) from the cartridge.

## Revendications

1. Seringue (1) pour le dosage unique d'une masse de remplissage (15), comportant
une chambre de stockage (5, 6, 7) pour recevoir la masse de remplissage (8, 9, 15), la chambre de stockage (5, 6, 7) comportant une extrémité de sortie (28) pour extraire la masse de remplissage (8, 9, 15) et une extrémité de transport (29) opposée à l'extrémité de sortie (28), la chambre de stockage (5, 6, 7) comprenant un élément de sortie obturable (13), de sorte que la masse de remplissage (8, 9, 15) peut être stockée dans la chambre de stockage (5, 6, 7), la chambre de stockage (5, 6, 7) comportant une première chambre partielle (6) qui contient une première composante capable de s'écouler (8), et une seconde chambre partielle (7) qui contient une seconde composante capable de s'écouler (9), la chambre de stockage (5, 6, 7) étant entourée par un boîtier (34),
un mélangeur (31) et un boîtier de mélangeur (42) qui sont montés à l'extrémité de sortie (28), le boîtier de mélangeur constituant l'élément de sortie obturable (13), le mélangeur (31) étant réalisé d'un seul tenant avec le boîtier (34) comprenant les première et seconde chambres partielles (6, 7), et le boîtier de mélangeur (42) étant relié au boîtier (34), et un élément d'accrochage (10) pour relier la chambre de stockage (5, 6, 7) à une cartouche (2) pour remplir la chambre de stockage (5, 6, 7) avec la masse de remplissage (8, 9, 15),
dans lequel
l'élément d'accrochage (10) comprend une première extrémité d'entrée (11) pour relier la première chambre partielle (6) à la cartouche (2), et une seconde extrémité d'entrée (12) pour relier la seconde chambre partielle (7) à la cartouche (2),
la première extrémité d'entrée (11) est réalisée sous la forme d'un premier manchon tubulaire (16) et la seconde extrémité d'entrée (12) est réalisée sous la forme d'un second manchon tubulaire (17) qui est destiné à être relié à la cartouche (2),
l'élément d'accrochage (10) comprend une saillie (37) qui relie les première et seconde chambres partielles (6, 7) l'une à l'autre,
le premier manchon tubulaire (16) comprend un premier canal (18) et le second manchon tubulaire (17) comprend un second canal (19), le premier canal étant relié à la première chambre partielle (6) et le second canal (19) étant relié à la seconde chambre partielle (7),
la seringue (1) comprend un élément d'extraction (3, 4, 30) qui peut être logé dans la chambre de stockage (5, 6, 7) pour extraire la masse de remplissage hors de la chambre de stockage (5, 6, 7),
l'élément d'extraction (30) comprend au moins un premier piston (3) et un second piston (4), le premier piston (3) pouvant être logé de façon mobile dans la première chambre partielle (6) et le second piston (4) pouvant être logé de façon mobile dans la seconde chambre partielle (7), de sorte que lors d'un mouvement de l'un au moins parmi le premier ou le second piston (3, 4), les première et seconde composantes capables de s'écouler (8, 9) peuvent être extraites simultanément, et il est prévu un poinçon (27), les premier et second pistons (3, 4) étant mobiles au moyen du poinçon (27) et le poinçon (27) étant réalisé d'un seul tenant avec le premier piston (3) et avec le second piston (4).

2. Seringue (1) selon la revendication 1,
dans laquelle
les premier et second manchons tubulaires (16, 17) sont reliés l'un à l'autre par un élément formant âme (45).

3. Seringue (1) selon l'une des revendications précédentes,
dans laquelle
la première chambre partielle (6) et la seconde chambre partielle (7) sont réalisées sous la forme d'un premier et d'un second tube (46, 47), les deux tubes (46, 47) étant reliés l'un à l'autre par l'élément d'accrochage (10).

4. Seringue (1) selon la revendication 3,
dans laquelle
l'un au moins des tubes (46, 47) comprend une zone (48, 49) qui présente un diamètre intérieur inférieur à celui du tube correspondant (46, 47).

5. Seringue (1) selon l'une des revendications 1 à 4,
dans laquelle
l'un au moins des manchons tubulaires (16, 17) comprend une zone (50, 51) qui présente un diamètre intérieur inférieur à celui du manchon tubulaire correspondant (16, 17).

6. Seringue (1) selon la revendication 2,
dans laquelle
l'élément formant âme (45) se raccorde à la saillie (37) et s'étend en direction des manchons tubulaires (16, 17).

7. Seringue (1) selon l'une des revendications précédentes,
dans laquelle
la chambre de stockage (5, 6, 7) est au moins partiellement transparente de manière à permettre de contrôler le niveau de remplissage de la masse de remplissage (8, 9, 15) dans la chambre de stockage (5, 6, 7).

8. Équipement de dosage d'une masse de remplissage (8, 9, 15), comportant une cartouche (2) et au moins une seringue (1) selon l'une des revendications précédentes,
dans lequel
la cartouche contient la masse de remplissage (15), et il est prévu un élément de sortie (22, 23) sur la cartouche, qui est prévu pour l'engagement dans l'élément d'accrochage (10) pour remplir la chambre de stockage (5, 6, 7) avec la masse de remplissage (8, 9, 15) depuis la cartouche.
